# EUROPEAN PATENT SPECIFICATION

(11) **EP 1 825 803 B1**
(45) Date of publication and mention of the grant of the patent: **08.04.2009**
(21) Application number: 07003888.0
(22) Date of filing: 26.02.2007
(51) Int. Cl.: A61B 1/00, A61B 17/04, A61B 17/28

(54) **Cap installable on distal end portion of endoscope**
Kappe zum Anbringen am distalen Ende eines Endoskops
Bouchon pouvant être installé sur la partie d'extrémité distale d'un endoscope

(30) Priority: 28.02.2006 US 363641
(43) Date of publication of application: 29.08.2007
(73) Proprietor: OLYMPUS MEDICAL SYSTEMS CORP., Tokyo (JP)
(72) Inventor: Mikkaichi, Takayasu, Shibuya-ku Tokyo (JP)
(74) Representative: von Hellfeld, Axel

(56) References cited:
- JP-A- 9 066 019
- JP-A- 59 093 413
- JP-A- 2004 000 601
- US-A1- 2004 127 767

## Description

### BACKGROUND OF THE INVENTION

### Field of the Invention

The present invention is related to a cap used by being installed on a distal end portion of an endoscope that is inserted into a living body through a natural orifice. The features of the preamble of independent claim 1 are known from JP-A-59 093 413.

### Description of Related Art

In recent years, various medical procedures have come to be carried out in a body cavity by using an endoscope. An example of a medical procedure is passing endoscopic suturing instruments through an endoscope when suturing tissues in a body cavity. Endoscopic suturing instruments are known that have suturing needles supported so as to be able to advance and retract in a sheath that is extended in a tube shape. The suturing needles have a tubular shape, and elongated anchors connected to suturing threads are inserted into the hole in the suturing needles.

While grasping forceps hold a portion of a thin tissue, the suturing needles are advanced to penetrate the tissue. When a T-bar is delivered from the distal end of a suturing needle while the suturing needle penetrates the tissue, the anchor and T-bar are retained on the outer side of the tissue. When the suturing needles are pulled out, the suturing thread passes through the tissue, and thereby when the suturing thread is pulled tight, the tissue is sutured by the suturing thread, which is prevented from slipping out by the anchor.

Here, suturing may be carried out by passing the endoscope and the endoscopic suturing instruments through an overtube having an opening formed in the side portion of the distal end thereof. At this time, the tissue is held by extending grasping forceps from the overtube after they have been passed through the opening on the side portion of the overtube. When the grasping forceps are pulled back while the tissue is being held, the tissue is drawn into the overtube from the opening. While the tissue remains held by the grasping forceps, the suturing needle is advanced to penetrate the tissue.

However, conventionally, because the force by which the suturing needles penetrate the tissue acted such that the tissue could be displaced, the penetration was not easy. When an overtube was used, because the opening of the overtube has a large opening area in order to insert and remove the forceps, it was not possible to prevent movement of the tissue when penetrating the tissue.

Endoscopic capsules adapted for selective attachment to the distal end portion of an endoscope are disclosed in JP-A-59 093 413 and US-A-2004/127 767. These capsules include a tissue access opening along a portion of the length of the capsules in order to allow a medical instrument to access patient tissue therethrough.

### SUMMARY OF THE INVENTION

An object of the present invention is to provide a device that facilitates a tissue puncturing operation using an endoscope.

In order to achieve the above object, the present invention provides a cap installable on a distal end portion of an endoscope, including: a cap body that is constituted by a hollow member having a distal end opening and a proximal end opening, and has a proximal end portion to be installed on the distal end portion of the endoscope; a first slit that is formed in the cap body and into which tissue is to be drawn, and that extends in a first direction from the distal end opening; and a second slit that is formed in the cap body and into which tissue is to be drawn, and that is connected to the first slit and extends in a second direction differing from the first direction.

The above cap may further include a movement restricting portion that is formed on the cap body between the second slit and the distal end opening, and the tissue that has been drawn into the second slit being restricted from moving toward the distal end opening.

The above cap may further include a guide portion that is formed in the cap body and that guides the tissue from the first slit to the second slit.

In the above cap, the first direction may be a longitudinal direction from the distal end opening to the proximal end opening of the cap body, and the second direction may be a circumferential direction perpendicular to the longitudinal direction of the cap body.

The cap body may have substantially cylindrical shape.

According to the present invention, when the tissue is drawn into the slit of the cap, the tissue does not move in the penetration direction; therefore, the tissue can be reliably penetrated. Because the first slit into which the tissue is drawn and the second slit that prevents the movement of the tissue are separate, the tissue is reliably penetrated when the suturing needle is advanced in the insertion direction.

### BRIEF DESCRIPTION OF THE DRAWINGS

FIG. 1 is a drawing in which a cap has been installed on a distal end of an endoscope.
FIG. 2 is a drawing showing the cap, and is a plan view along the arrow A in FIG. 1.
FIG. 3 is a cross-sectional view of the distal end portion of a suturing device.
FIG. 4 is a drawing showing the structure of a retaining device.
FIG. 5 is a drawing for explaining a procedure in which the circumferential edge of a tissue opening portion is grasped by grasping forceps of the suturing device.
FIG 6 is a drawing in which the tissue that has been grasped by the grasping forceps of the suturing device is passed through a first slit and is drawn into the cap.
FIG. 7 is a drawing in which the endoscope has been rotated, the tissue has been guided into a second slit, and suturing needles have penetrated the tissue.
FIG. 8 is a drawing in which the suturing needles have been withdrawn and the suturing thread has been passed through the tissue.
FIG 9 is a drawing for explaining the structure and method of use of the fastening tool.
FIG 10 is a drawing in which the tissue opening portion has been sutured by the retaining device.
FIG 11 is a drawing in which the cap has been installed on an endoscope having two channels.
FIG 12 is a plane view showing the shape of another cap.
FIG 13 is a plane view showing the shape of another cap.

### DETAILED DESCRIPTION OF THE INVENTION

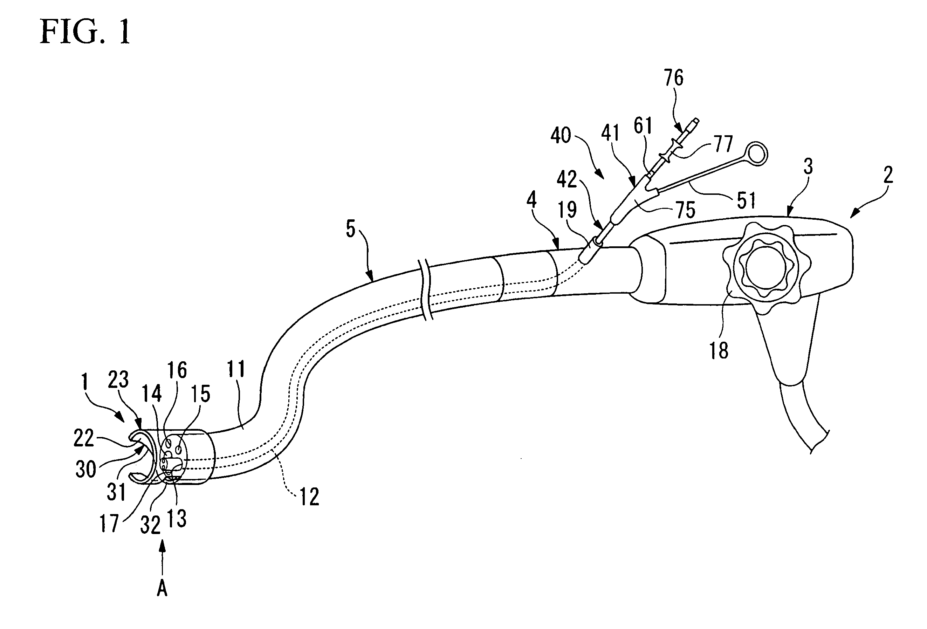

FIG. 1 shows an endoscope 2 on which the cap 1 has been installed. The endoscope 2 has: a control portion 3 that is held and operated by an operator; and a flexible insertion portion 5, which is inserted into a body cavity through a connecting portion 4, that extends from the distal end of this control portion 3.

The insertion portion 5 has, at the distal end thereof, a bending portion 11 that carries out the bending action. A working channel 12 is provided in the insertion portion 5. A distal end opening portion 13 of the working channel 12 is formed on a distal end surface 9 of the insertion portion 5. An observation apparatus 14 and an illumination apparatus 15, which are used when capturing observation images inside the living body, are provided in proximity to the distal end opening portion 13. In addition, a distal end opening portion for an air and water supplying channel 16 and a distal end opening portion for a suction channel 17 are also provided.

A control knob 18 is provided in the control portion 3. The bending portion 11 of the insertion portion 5 is connected to the control knob 18 by a wire (not illustrated), and by controlling the control knob 18, the bending portion 11 is bent in the vertical and lateral directions, and thereby the distal end portion 9 thereof can be oriented in the desired direction.

In addition, an insertion opening 19 is provided on the side portion of the connecting portion 4, and the insertion opening 19 is connected to the distal end opening portion 13 via the working channel 12.

The cap 1 installed on the distal end of the insertion portion 5 has a substantially cylindrical cap body 23 having a proximal end opening 21 and a distal end opening 22. An annular rib 24 is formed on the inner periphery of the cap body 23 between the proximal end opening 21 and the distal end opening 22. A portion of the cap 1 which is closer to the proximal end opening 21 than from the rib 24 serves as a connecting portion 25 (proximal end portion) that can engage the outer periphery of the distal end of the insertion portion 5, and a distal end portion 26 which is closer to the distal end opening 22 than from the rib 24 projects farther from the distal end of the endoscope 2.

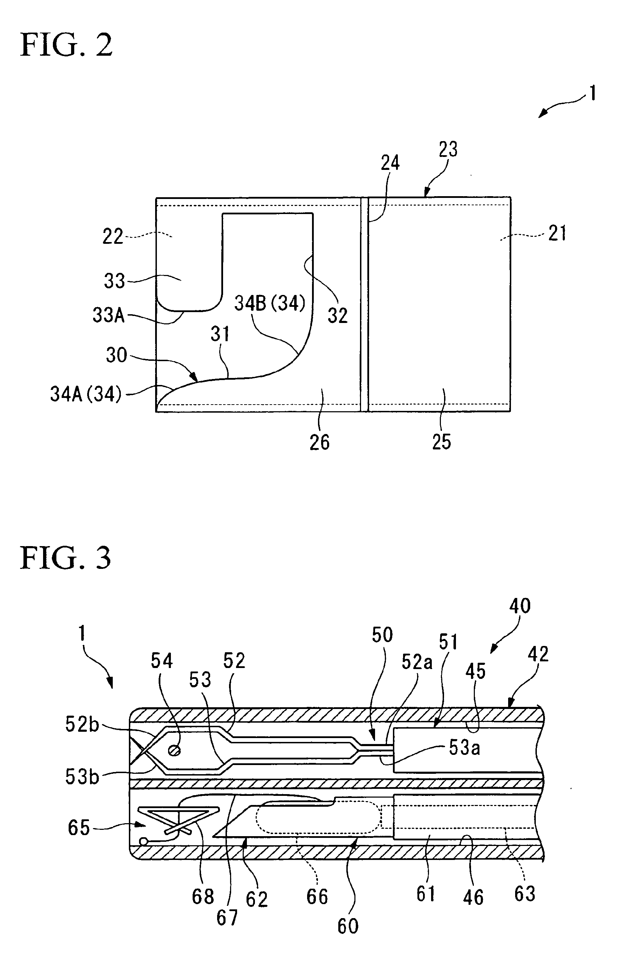

As shown in FIG. 1 and FIG. 2, a substantially L-shaped slit 30 that passes from the outer circumferential surface to the inner circumferential surface is formed in the distal end portion 26. The slit 30 has a first slit 31 (middle opening portion) that extends from the distal end opening 22 along a substantially axial direction (first direction) of the cap body 23, and a second slit 32 (side opening portion) that extends in the circumferential direction (second direction) from the proximal end side of the first slit 31. Due to the slit 30, a tongue shaped tissue movement restricting portion 33 that extends in the circumferential direction is formed on the circumferential edge portion of the distal end opening 22 of the cap body 23. The corner portion of the end portion 33A facing the first slit 31 of the tissue movement restricting portion 33 is cut into a curved surface shape. Furthermore, a guiding portion 34 that has a curved surface shape facing the second slit 32 is provided on the first slit 31. The guiding portion 34 has a curved surface portion 34A and curved surface portion 34B. In the curved surface portion 34A, the distal end opening 22 is greatly slanted such that the width of the first slit 31 in the circumferential direction increases in the direction of separation from the tissue movement restricting portion 33. In the curved surface portion 34B, the proximal end portion faces the second slit 32 and the width of the first slit 31 in the circumferential direction smoothly decreases towards the second slit 32. Note that either the distal end portion 26 or the entirety of the cap 1 is manufactured from a transparent material so that the field of view of the observation apparatus 14 in the endoscope 2 is not obstructed. Furthermore, preferably the cap 1 is manufactured from an elastically deformable resin in order to facilitate the installation on and release from the endoscope 2.

Here, as shown in FIG. 1, the suturing device 40, which is an endoscopic instrument used by being passed through the working channel 12 of the endoscope 2, will be explained. The suturing device 40 has a control portion 41 that is controlled by an operator, and a long and flexible supporting sheath 42, which extends from the control portion 41, passes through the working channel 12 so as to freely advance and retract.

As shown in FIG. 3, the cross-section of the supporting sheath 42, which is perpendicular to the longitudinal direction, is circular, and a grasping forceps insertion channel 45 and a needle insertion channel 46 are formed parallel to the longitudinal direction. Grasping forceps 50 pass through the grasping forceps insertion channel 45. The grasping forceps 50 have a long and flexible advance-retract control member 51, and the proximal end portions of a pair of holding pieces 52 and 53 are fastened to the distal end of the advance-retract control member 51. The proximal end portions of the pair of holding pieces 52 and 53 serve as abutting portions 52a and 53a, which abut each other when the holding pieces 52 and 53 are closed. The distal end portions of the holding pieces 52 and 53 have a hooked shape. When the holding pieces 52 and 53 inside the distal end portion of the grasping forceps insertion channel 45 are drawn, the holding pieces 52 and 53 close such that the distal end portions 52b and 53b intersect. Here, in proximity to the distal end portion of the grasping forceps insertion channel 45, a pin 54 is provided perpendicular to the advancing and retracting direction of the grasping forceps 50. The pin 54 is disposed between the distal end portions 52b and 53b, which intersect the holding pieces 52 and 53, and the abutting portions 52a and 53a. Therefore, when the advance-retract control member 51 advances with respect to the supporting sheath 42 and the pair of holding pieces 52 and 53 project from the distal end of the supporting sheath 42, the pin 54 enters between the abutting portions 52a and 53a, and the holding pieces 52 and 53 are both spread open.

In the needle device 60, a hollow suturing needle 62 is fastened to the distal end of the long and flexible tube 61. The distal end of the suturing needle 62 is a sharp pointed portion that is slanted at an acute angle. A pusher 63 passes through the tube 61 and the suturing needle 62 so as to freely advance and retract.

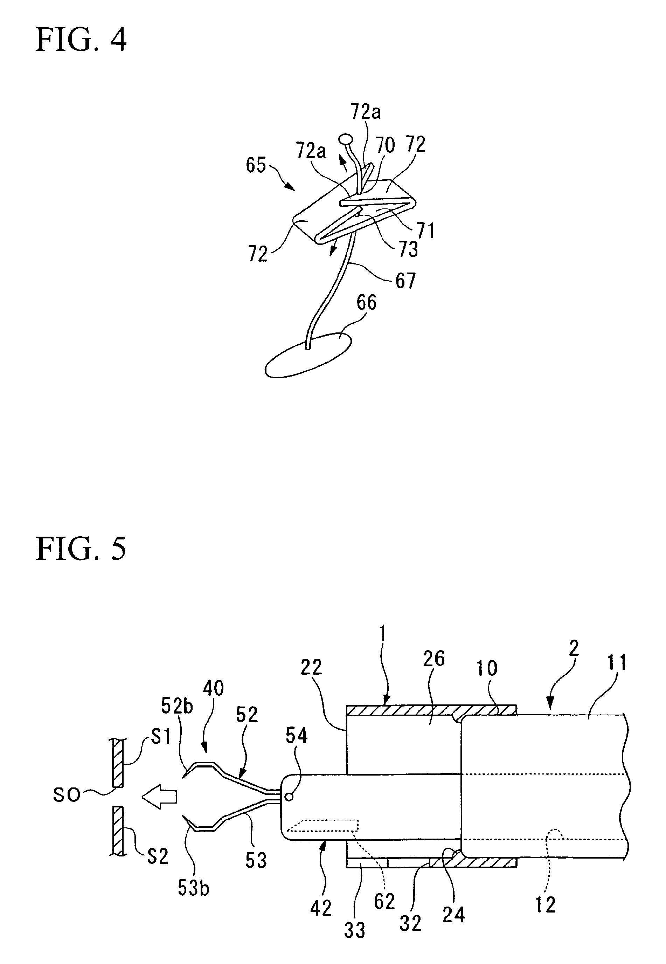

Here, an anchor 66 of the retaining device 65 is accommodated in the suturing needle 62 more toward the distal end side than the pusher 63. As shown in FIG. 3 and FIG. 4, the retaining device 65 has the anchor 66 that is attached to the suturing thread 67 and a lock member 68. The anchor 66 is a columnar member, and the proximal portion of the suturing thread 67, which passes through the living body tissue, is engaged at the center area in the longitudinal direction thereof. The lock member 68 functions to prevent the suturing thread 67 from falling out after the suture has been completed. The lock member 68 includes a proximal portion 71 that extends in a plate shape and two folding pieces 72 that rise from both ends of the proximal portion 71 in the longitudinal direction so as to face each other at a prescribed angle. The proximal portion 71 and the folding pieces 72 are formed so as to be integrally linked by an elastic member. An opening portion 73 is formed at the center of the proximal portion 71. Projecting pieces 72a are provided on the distal ends of the folding pieces 72, and both of these projecting pieces 72a are engaged together. In addition, the suturing thread 67 is passed in sequence through the opening portion 73 of the proximal portion 71 and the engaging portion 70 formed by both projecting pieces 72a, and a retainer by a knot or melting treatment is formed in a distal end of the suturing thread 67.

Note that when the suturing thread 67 is pulled from the back end towards the distal end while the lock member 68 is fastened, the engaging portion 70 expands due to both folding pieces 72 elastically deforming in a spreading direction, and thereby the suturing thread 67 moves towards the distal end side. In contrast, when the suturing thread 67 is pulled from the distal end towards the back end, the engaging portion 70 contracts due to both folding pieces 72 elastically deforming in the direction of closure, and thereby the movement of the suturing thread 67 is restricted. Specifically, when the suturing thread 67 is pulled towards the distal end, the lock member 68 moves in a direction approaching the anchor 66. However, because the movement of the lock member 68 in the direction of separation from the anchor 66 is restricted, the state in which both of the anchor 66 and the lock member 68 approach each other is maintained.

Note that, as shown in FIG. 1, the control portion 41 of the suturing device 40 has a control body 75 to which the proximal end portion of the supporting sheath 42 is fastened. The advance-retract control member 51 of the holding forceps 50 is pulled out of the control body 75, and can carry out the advancing and retracting action. Furthermore, the tube 61 of the needle device 60 is pulled out of the control body 75, and can carry out the advancing and retracting action. A needle device control portion 76 is provided on the proximal end portion of the tube 61. The needle device control portion 76 has a slider 77, and a pusher 63 can carry out the advancing and retracting action.

Next, the operation of this embodiment will be explained. In the following, an example will be explained in which a tissue opening formed in the tissue in a body cavity is closed by a suture.

First, the cap 1 is installed on the distal end of the endoscope 2, and the suturing device 40 is passed through the working channel 12 of the endoscope 2. At this time, the holding forceps 50 are accommodated in the holding forceps insertion channel 45, and the needle device 60 is accommodated in the needle insertion channel 46. The retaining device 65 is accommodated inside the suturing needle 62 and the needle insertion channel 46. The orientation of the suturing device 40 with respect to the endoscope 2 is adjusted such that the suturing needle 62 and the holding forceps 50 are disposed in sequence from the first slit 31 of the cap 1.

The operator inserts the endoscope 2 into the living body while observing observation images of the inside of the living body captured by using the observation apparatus 14 and the illumination apparatus 15 using a monitor (not illustrated). As shown in FIG. 5, when the distal end of the endoscope 2 has been guided into proximity to the tissue opening SO, the tissue pieces S 1 and S2, which are at opposing locations on the circumferential edge portion of the tissue opening SO, are identified by using the observation images captured by the observation apparatus 14, and the position and the orientation of the endoscope 2 are adjusted such that the axial line of the insertion portion 5 is substantially perpendicular to the longitudinal direction of the tissue opening SO.

The operator advances the suturing device 40 and projects the distal end of the supporting sheath 42 from the distal end opening 22 of the cap 1. Then, the operator advances the advance-retract control member 51 while keeping the supporting sheath 42 stationary, and opens the pair of holding pieces 52 and 53 at a position projecting beyond the cap 1. The bending portion 11 of the endoscope 2 is bent, the tissue piece S 1 is pressed against the distal end portion 52b of one holding piece 52, and the tissue piece S2 is pressed against the distal end portion 53b of the other holding piece 53. When the advance-retract control member 51 is retracted while keeping the supporting sheath 42 stationary, the pair of holding pieces 52 and 53 close, and the tissue pieces S 1 and S2 are held so as to be interposed between the holding pieces 52 and 53.

While the tissue pieces S1 and S2 remain held by the holding pieces 52 and 53, the entire suturing device 40 is pulled back, and retracted into the cap 1. Thereby, the tissue pieces S1 and S2 (and the tissue opening SO) are pulled, and the tissue wall SW formed at this time is pulled into the first slit 31 of the cap 1. Note that instead of pulling back the suturing device 40, the endoscope 2 may be advanced relatively while the position of the suturing device 40 is fixed.

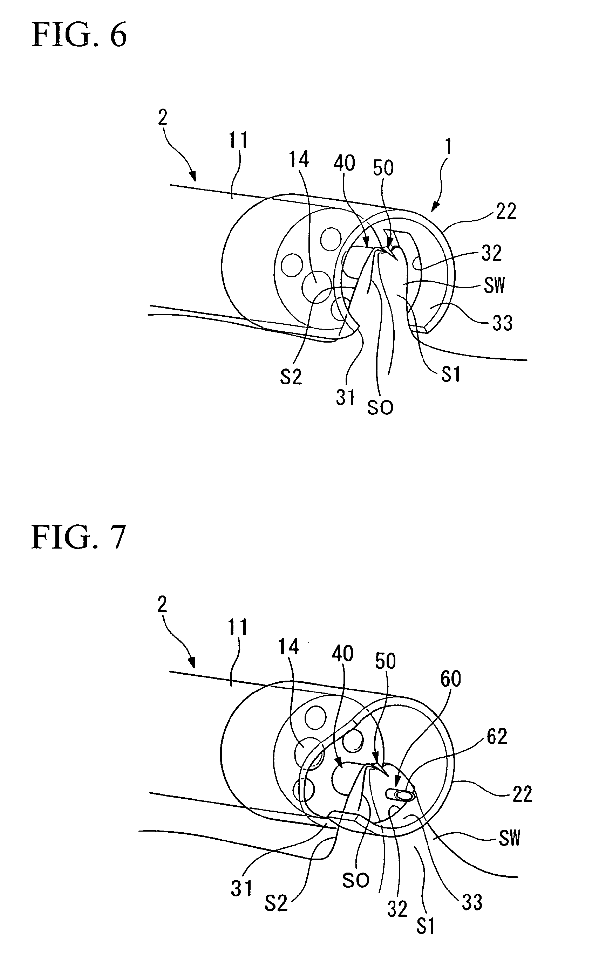

Next, the endoscope 2 is rotated around the axial line. As shown in FIG. 7, the tissue wall SW is thereby inserted into the second slit 32.
At this time, the tissue wall SW is smoothly guided from the first slit 31 to the second slit 32 by the guide portion 34.

After the tissue wall SW has entered the second slit 32, in this state the suturing needle 62 is advanced with respect to the supporting sheath 42. The lock member 68 is pressed and the front side of the tissue wall SW is lowered into the cap 1. The suturing needle 62 that projects from the supporting sheath 42 punctures the front side of the tissue wall SW. At this time, the outer side of the tissue wall SW abuts the tissue movement restricting portion 33 and prevents the movement of the tissue wall SW. Thereby, when the suturing needle 62 advances further, the suturing needle 62 penetrates in sequence the tissue piece S2, the tissue opening SO and the tissue piece S1, and passes out of the outer side of the tissue wall SW.

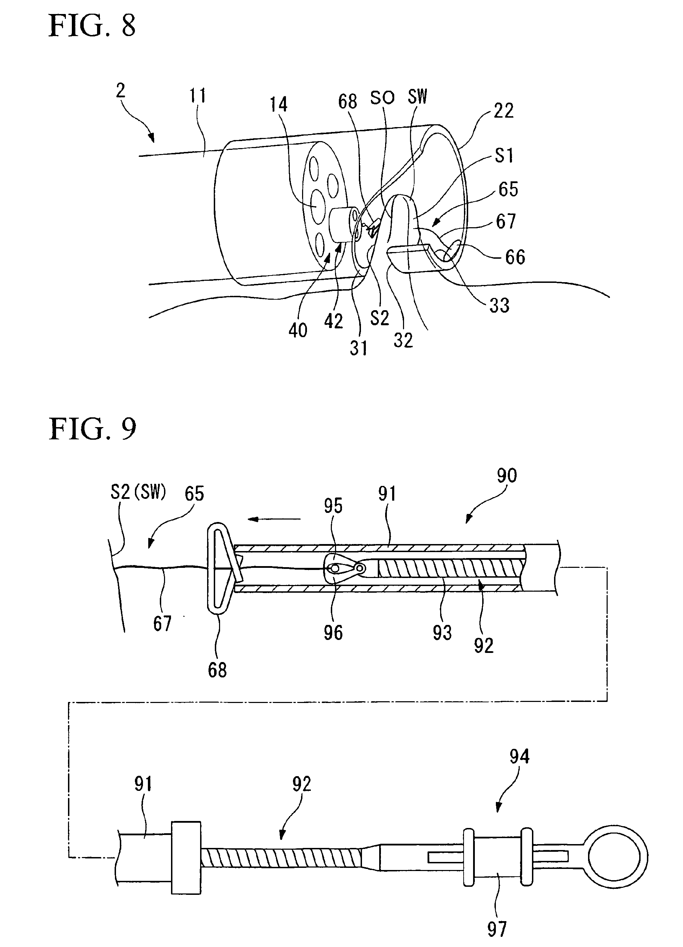

The slider 77 of the control portion 41 of the suturing device 40 is advanced, and thereby the pusher 63 presses the anchor 66 out from the suturing needle 62 to the outer side of the tissue wall SW. Subsequently, the suturing needle 62 is pulled back and extracted from the tissue wall SW, and then accommodated in the support sheath 42. As shown in FIG 8, the suturing thread 67 engaged by the anchor 66 retained in the outer side of the tissue wall SW remains passing through the tissue piece S2 and the tissue piece S 1. Because the anchor 66 is disposed on the outer side of the tissue wall SW by the suturing thread 67 and the lock member 68 is disposed on the proximal side, the suturing thread 67 is tightened such that the lock member 68 is pressed against the tissue piece S2, and thereby the tissue opening SO is sutured.

Instead of the suturing device 40, for example, the fastening tool 90 shown in FIG. 9 may be passed through the working channel 12. The fastening tool 90 provides a fastening sheath 91 that extends in a tube shape and a forceps portion 92 that passes through the inside of this fastening sheath 91. The forceps 92 have a forceps sheath 93, and the distal end of the forceps sheath 93 is pulled from the proximal end of the fastening sheath 91 to connect to the control portion 94. A pair of forceps pieces 95 and 96 is provided so as to freely open and close on the distal end portion of the forceps sheath 93. The pair of forceps pieces 95 and 96 can be opened and closed by the slider 97 of the control portion 94.

After the distal end of the suturing thread 67 is held by opening and closing the forceps pieces 95 and 96, the fastening sheath 91 is advanced. The fastening sheath 91 goes past the forceps pieces 95 and 96 to abut the lock member 68. Then, the fastening sheath 91 is advanced and the lock member 68 presses against the front side (the tissue piece S2) of the tissue wall SW. Because the lock member 68 can move in the direction approaching the anchor 66, by advancing the fastening sheath 91, the tissue pieces S1 and S2 are in close contact due to being interposed between the lock member 68 and the anchor 66, and thereby the tissue opening SO is closed. Note that because the movement of the lock member 68 is restricted in the direction of separation from the anchor 66, the closure of the tissue opening SO can be maintained.

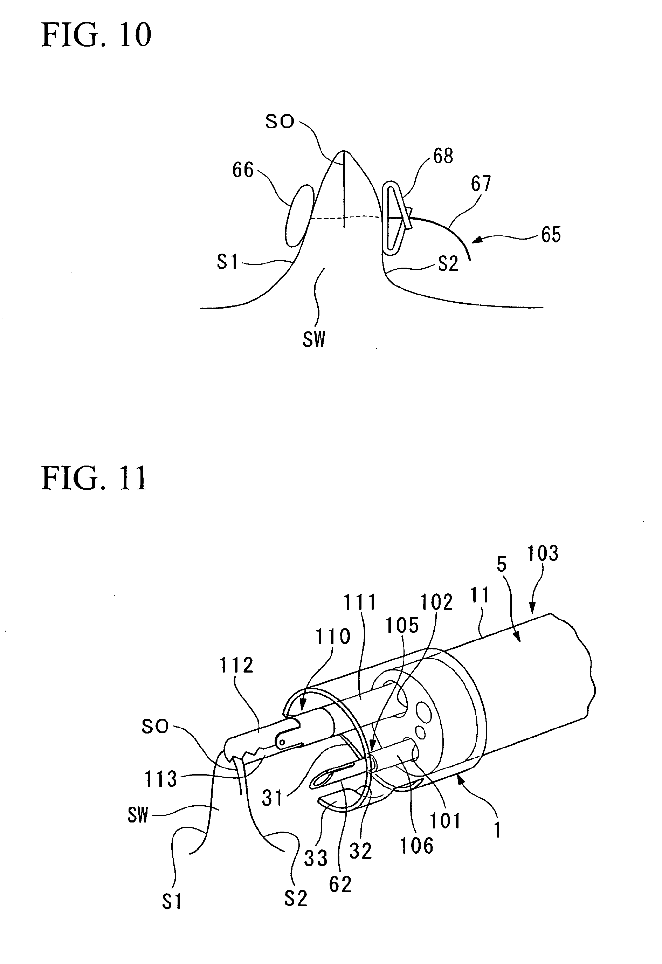

After the suturing of the tissue opening SO has been completed, the endoscope 2 is rotated around its axial line. After the tissue wall SW has moved from the second slit 32 to the first slit 31, the endoscope 2 is retracted. The tissue wall SW separates from the inside of the cap 1 by passing through the first slit 30. As shown in FIG 10, tissue opening SO sutured by the retaining device 65 remains. When the endoscope 2 is removed from the body cavity, this sequence of processes ends.

In the present embodiment, the tissue is drawn into the slit 30 of the cap 1, and the tissue does not move in the penetration direction. Conventionally, because the force by which the suturing needles 62 penetrate the tissue acted such that the tissue could be displaced, the penetration was not easy. However, in this embodiment, because the tissue drawn into the slit 30 cannot move, the tissue can be reliably penetrated.

Because the opening (the first slit 31) into which the tissue is drawn and the opening (the second slit 32) that prevents the movement of the tissue are separate, when the suturing needle 62 is advanced in the insertion direction, the tissue is reliably penetrated. Conventionally, because the opening of the overtube has a large opening area in order to insert and remove the forceps, it was not possible to prevent movement of the tissue when penetrating the tissue. However, the movement of the tissue can be reliably prevented by using this cap 1. Furthermore, because a guide portion 34 is provided in the first slit 31, the tissue can be easily guided from the first slit 31 to the second slit 32.

Because the slit 30 of the cap 1 is substantially L-shaped, it is possible to draw the tissue by an advancing and retracting action and a rotating action that are frequently used in normal medical procedures, and thereby control is simplified for the operator.

When the length of the tissue movement restricting portion 33 of the cap 1 in the insertion direction is set to a length at which the suturing needle 62 does not project from the distal end opening 22 of the cap 1, the suturing needle 62 does not penetrate other tissue when the tissue is penetrated, and the manipulation is simplified. In addition, by providing stoppers in the suturing device 40 and mechanically limiting the amount of the projection of the suturing needle 62, it is possible to prevent more reliably the projection of the suturing needle 62.

Because there is a tissue opening SO, when executing suturing in an organ that is withered and cannot be distended, both the field of view of the observation apparatus 14 and the working space can be ensured since the organ is spread apart by the cap 1.

Here, a modification of the present embodiment will be explained.

As shown in FIG 11, the cap 1 may be installed on what is called a multi-channel endoscope 103, which has two or more working channels 101 and 102. At this time, the suturing device 105 is structured such that the suturing device 105 is used by being passed through one working channel 101 so as to freely advance and retract, and the suturing needle 62 can be passed through the protecting sheath 106 so as to freely advance and retract. Furthermore, a retaining device 65 is accommodated inside the suturing needle 62 and the distal end portion of the protecting sheath 106. The grasping forceps 110 are passed through the other working channel 102. The grasping forceps 110 have a structure in which a pair of holding pieces 112 and 113 is passed through the sheath 111 so as to freely advance and retract, and the pair of holding pieces 112 and 113 are opened and closed by the operation on the proximal side. In this manner, even when the cap 1 is installed on the endoscope 103, effects identical to those described above can be attained.

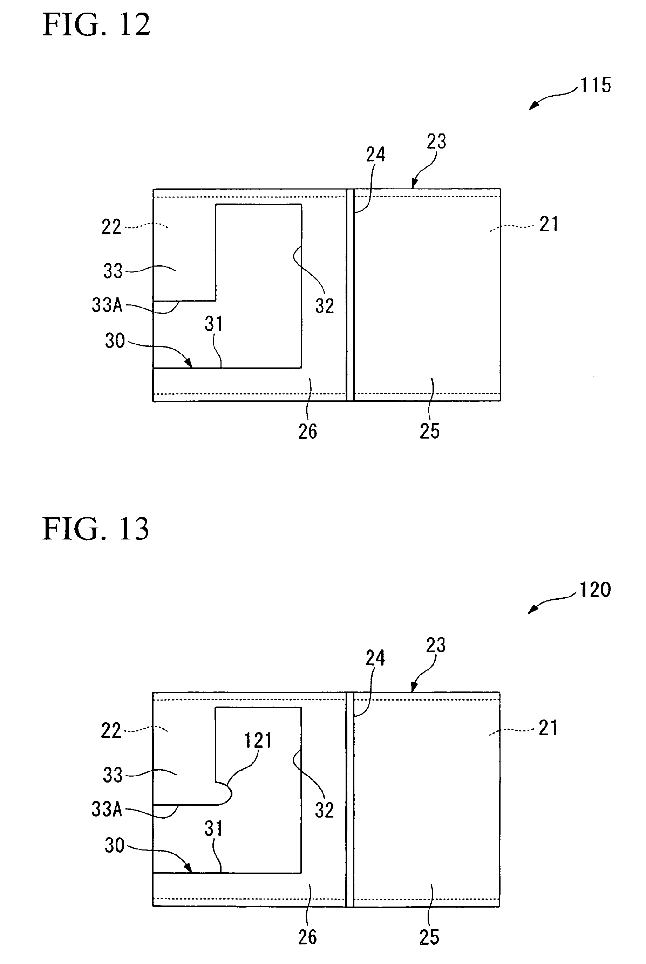

In addition, the cap 115 shown in FIG. 12 may have an L shape without a guide portion 34. In addition to the effects described above, this cap is easily manufactured. The corner of the end portion 33A of the tissue movement restricting portion 33 may be cut so as to have a curved surface shape.

In the tissue movement restricting portion 33, the cap 120 shown in FIG 13 may provide a projection 121 in order to prevent slippage on the end portion 33A facing the first slit 31. The projection 121 has a curved surface shape extending towards the proximal side. When such a projection 121 is provided, the tissue that has been drawn into the second slit 32 is displaced with difficulty in the first slit 31, and the tissue can be more reliably and easily penetrated.

The first slit 31 may be formed in a direction (first direction) intersecting the axial direction. The second slit 32 may be formed in a direction (second direction) intersecting the circumferential direction.

While preferred embodiments of the invention have been described and illustrated above, it should be understood that these are exemplary of the invention and are not to be considered as limiting. Additions, omissions, substitutions, and other modifications can be made without departing from the spirit or scope of the present invention. Accordingly, the invention is not to be considered as being limited by the foregoing description, and is only limited by the scope of the appended claims.

## Claims

1. A cap installable on a distal end portion of an endoscope, comprising:
a cap body (23) that is constituted by a hollow member having a distal end opening (22) and a proximal end opening (21), and has a proximal end portion (25) to be installed on the distal end portion of the endoscope;
a first slit (31) that is formed in the cap body (23) and into which tissue is to be drawn, and that extends in a first direction from the distal end opening (22);
**characterized by**
a second slit (32) that is formed in the cap body (23) and into which tissue is to be drawn, and that is connected to the first (31) slit and extends in a second direction differing from the first direction.

2. The cap installable on a distal end portion of an endoscope according to claim 1, further comprising:
a movement restricting portion (33) that is formed on the cap body (23) between the second slit (32) and the distal end opening (22), and the tissue that has been drawn into the second slit (32) being restricted from moving toward the distal end opening (22).

3. The cap installable on a distal end portion of an endoscope according to claim 1 or 2, further comprising:
a guide portion (34) that is formed in the cap body (23) and that guides the tissue from the first slit (31) to the second slit (32).

4. The cap installable on a distal end portion of an endoscope according to any one of claims 1 to 3, wherein the first direction is a longitudinal direction from the distal end opening (22) to the proximal end opening (21) of the cap body (23), and the second direction is a circumferential direction perpendicular to the longitudinal direction of the cap body (23).

5. The cap installable on a distal end portion of an endoscope according to any one of claims 1 to 4, wherein:
the cap body (23) has a substantially cylindrical shape.

## Patentansprüche

1. An einem distalen Endabschnitt eines Endoskops anbringbare Kappe, umfassend:
einen Kappenkörper (23), der durch ein Hohlelement mit einer distalen Endöffnung (22) und einer proximalen Endöffnung (21) gebildet ist, und einen proximalen Endabschnitt (25) aufweist, der an dem distalen Endabschnitt des Endoskops anzubringen ist;
einen ersten Schlitz (31), der in dem Kappenkörper (23) ausgebildet ist und in welchen Gewebe einzuziehen ist, und der sich in eine erste Richtung von der distalen Endöffnung (22) erstreckt;
**gekennzeichnet durch**
einen zweiten Schlitz (32), der in dem Kappenkörper (23) ausgebildet ist und in welchen Gewebe einzuziehen ist, und der mit dem ersten (31) Schlitz verbunden ist und sich in eine von der ersten Richtung abweichende zweite Richtung erstreckt.

2. An einem distalen Endabschnitt eines Endoskops anbringbare Kappe nach Anspruch 1, weiter umfassend:
einen Bewegungsbegrenzungsabschnitt (33), der an dem Kappenkörper (23) zwischen dem zweiten Schlitz (32) und der distalen Endöffnung (22) ausgebildet ist, und wobei das Gewebe, das in den zweiten Schlitz (32) eingezogen worden ist, davon abgehalten wird, sich in Richtung der distalen Endöffnung (22) zu bewegen.

3. An einem distalen Endabschnitt eines Endoskops anbringbare Kappe nach Anspruch 1 oder 2, weiter umfassend:
einen Führungsabschnitt (34), der in dem Kappenkörper (23) ausgebildet ist und der das Gewebe von dem ersten Schlitz (31) zu dem zweiten Schlitz (32) führt.

4. An einem distalen Endabschnitt eines Endoskops anbringbare Kappe nach einem der Ansprüche 1 bis 3, wobei die erste Richtung eine Längsrichtung von der distalen Endöffnung (22) zu der proximalen Endöffnung (21) des Kappenkörpers (23) ist, und die zweite Richtung eine Umfangsrichtung senkrecht zu der Längsrichtung des Kappenkörpers (23) ist.

5. An einem distalen Endabschnitt eines Endoskops anbringbare Kappe nach einem der Ansprüche 1 bis 4, wobei:
der Kappenkörper (23) eine im Wesentlichen zylindrische Form aufweist.

## Revendications

1. Embout installable sur une partie d'extrémité distale d'un endoscope, comprenant :
un corps d'embout (23) qui est constitué par un élément creux ayant une ouverture d'extrémité distale (22) et une ouverture d'extrémité proximale (21), et a une partie d'extrémité proximale (25) destinée à être installée sur la partie d'extrémité distale de l'endoscope ;
une première fente (31) qui est formée dans le corps d'embout (23) et dans laquelle un tissu est destiné à être tiré, et qui s'étend dans une première direction à partir de l'ouverture d'extrémité distale (22) ;
**caractérisé par**
une deuxième fente (32) qui est formée dans le corps d'embout (23) et dans laquelle un tissu est destiné à être tiré, et qui est connectée à la première fente (31) et s'étend dans une deuxième direction différente de la première direction.

2. Embout installable sur une partie d'extrémité distale d'un endoscope selon la revendication 1, comprenant en outre :
une partie de restriction de mouvement (33) qui est formée sur le corps d'embout (23) entre la deuxième fente (32) et l'ouverture d'extrémité distale (22), et le tissu qui a été tiré à l'intérieur de la deuxième fente (32) étant empêché de se déplacer vers l'ouverture d'extrémité distale (22).

3. Embout installable sur une partie d'extrémité distale d'un endoscope selon la revendication 1 ou 2, comprenant en outre :
une partie de guide (34) qui est formée dans le corps d'embout (23) et qui guide le tissu de la première fente (31) jusqu'à la deuxième fente (32).

4. Embout installable sur une partie d'extrémité distale d'un endoscope selon l'une quelconque des revendications 1 à 3, dans lequel la première direction est une direction longitudinale de l'ouverture d'extrémité distale (22) jusqu'à l'ouverture d'extrémité proximale (21) du corps d'embout (23), et la deuxième direction est une direction circonférentielle perpendiculaire à la direction longitudinale du corps d'embout (23).

5. Embout installable sur une partie d'extrémité distale d'un endoscope selon l'une quelconque des revendications 1 à 4, dans lequel :
le corps d'embout (23) a une forme substantiellement cylindrique.
